# EUROPÄISCHE PATENTSCHRIFT

(11) **EP 1 972 275 B1**
(45) Veröffentlichungstag und Bekanntmachung des Hinweises auf die Patenterteilung: **28.10.2015**
(21) Anmeldenummer: 07005637.9
(22) Anmeldetag: 20.03.2007
(51) Int. Cl.: A61B 5/151, A61B 5/1495

(54) **System zur in-vivo Messung einer Analytkonzentration**
System for in vivo measurement of an analyte concentration
Système destiné à la mesure in vivo d'une concentration d'un analyte

(43) Veröffentlichungstag der Anmeldung: 24.09.2008
(73) Patentinhaber: Roche Diagnostics GmbH, 68305 Mannheim (DE); F.Hoffmann-La Roche AG, 4070 Basel (CH)
(72) Erfinder: Roesicke, Bernd, 68305 Mannheim (DE); Rasch-Menges, Juergen, 68723 Schwetzingen (DE)
(74) Vertreter: Twelmeier Mommer & Partner

(56) Entgegenhaltungen:
- EP-A1- 1 683 484
- US-A1- 2006 142 651
- US-B1- 6 809 653

## Beschreibung

Die Erfindung betrifft ein System zur in-vivo Messung einer Analytkonzentration in einem menschlichen oder tierischen Körper, mit den im Oberbegriff des Anspruchs 1 angegebenen Merkmalen. Ein solches System ist aus den Patentschriften US 6809653 B1 und US 2006/0142651 A1 bekannt.

Sensoren zur Messung von Analytkonzentrationen von Körperflüssigkeiten, beispielsweise Blut oder interstitielle Flüssigkeit, lassen sich in der Regel nicht mit exakt vorgegebenen Messsensitivitäten produzieren. Typischerweise treten zwischen Produktionschargen erhebliche Schwankungen der Sensorsensitivität auf. Damit aus den Messsignalen von für in-vivo Messungen verwendeten Sensoren mit einer für medizinische Anwendungen hinreichenden Genauigkeit Analytkonzentrationen ermittelt werden können, sind deshalb Kalibrationsdaten erforderlich, die entweder an den betreffenden Sensor selbst oder mittels Stichproben an anderen Sensoren der betreffenden Produktionscharge ermittelt wurden. Derartige Kalibrationsdaten beschreiben im Allgemeinen den Unterschied zwischen einer idealen Sensorsensitivität und einer ermittelten Sensorsensitivität.

Systeme zur in-vivo Messung von Analytkonzentrationen umfassen in der Regel austauschbare Sensoren als Austausch- oder Verbrauchskomponenten und eine langlebige Basisstation, an welche die austauschbaren Sensoren angeschlossen werden. Daraus resultiert das Problem, dass dem System bei jedem Sensorwechsel neue Kalibrationsdaten zur Verfügung gestellt werden müssen.

Prinzipiell können diese Kalibrationsdaten auf einem Beipackzettel des Sensors zur Verfügung gestellt werden und von einem Benutzer von Hand in das System eingegeben werden. Da bei dieser Vorgehensweise jedoch die Gefahr von Eingabefehlern besteht, ist es günstiger, jedem Sensor oder jeder Sensorpackung einen Datenträger mit darauf gespeicherten Kalibrationsdaten beizufügen und so das Risiko einer fehlerhaften Eingabe auszuschließen.

Auch diese Lösung ist jedoch nicht perfekt, da die Gefahr besteht, dass zu verschiedene Sensoren gehörende Datenträger von Benutzern vertauscht werden und auf diese Weise einem System falsche Kalibrationsdaten zur Verfügung gestellt werden, die dann zu falschen Messergebnissen führen.

Aufgabe der Erfindung ist es deshalb, einen Weg aufzuzeigen, wie bei einem System der Eingangs genannten Art die Zuverlässigkeit erhöht und die Handhabung für einen Benutzer erleichtert werden kann.

Diese Aufgabe wird erfindungsgemäß gelöst durch eine Vorrichtung gemäß Anspruch 1.

Die erfindungsgemäße Maßnahme hat den Vorteil, dass für einen Benutzer das Anschließen eines Sensors an die Basisstation ebenso wie das Einspeisen der dazugehörenden Kalibrationsdaten erheblich vereinfacht wird. Sensor und Datenträger werden an die Basisstation in einem einzigen Arbeitsschritt angeschlossen, indem das Gehäuse, in dem sie gelagert sind, an eine an das Gehäuse angepasst Schnittstelle der Basisstation angesetzt wird. Der Gefahr einer Vertauschung von Datenträgern oder eines fehlerhaften Anschlusses der Sensoren kann auf diese Weise wirksam begegnet werden.

Bei dem Gehäuse, in dem mindestens ein Sensor und ein dazugehörender Datenträger mit Kalibrationsdaten gelagert sind, kann es sich um ein Verpackungsgehäuse handeln, das bestimmungsgemäß vor dem Durchführen einer in-vivo Messung ganz oder teilweise wieder von der Basisstation entfernt wird. Es ist aber auch möglich, dass das Gehäuse im Betrieb, also bei Durchführen von in-vivo Messungen, mit der Basisstation verbunden bleibt.

Auf dem Weg zu der beschriebenen Lösung mussten die Erfinder das folgende Problem lösen: Sensoren eines in-vivo Messsystems müssen steril sein, da sie in den Körper eines Patienten eingeführt werden. Bei gemeinsamer Verpackung mit einem Datenträger in einem einzigen Gehäuse ist jedoch die zur Sterilisation von Sensoren übliche Methode, nämlich intensive Bestrahlung, mit erheblichen Schwierigkeiten verbunden. Elektronische oder magnetische Datenträger werden nämlich durch die zur Sterilisation erforderliche Strahlendosis beeinträchtigt, so dass es nicht oder nur mit sehr teueren, speziell angefertigten Datenträgern möglich ist, das versiegelte Gehäuse mit darin angeordnetem Sensor und Datenträger zur Sterilisation des Sensors zu bestrahlen. Eine Verpackung der Sensoren nach erfolgter Sterilisation erfordert jedoch ebenfalls einen sehr hohen Aufwand, da dann bis zum Versiegeln des Gehäuses während des gesamten Verpackungsprozesses sterile Bedingungen gewahrt werden müssen.

Die erfindungsgemäße Lösung trotz dieser Probleme kostengünstig eine sterile Verpackung des Sensors mit dem dazugehörenden Datenträger zu ermöglichen, sieht ein Gehäuse mit mindestens zwei getrennten Kammern vor. Zum Verpacken eines Sensors und eines dazugehörenden Datenträgers wird zunächst der Sensor in der ersten Gehäusekammer angeordnet, die Gehäusekammer anschließend versiegelt und der Sensor in der ersten Gehäusekammer durch Strahlungseinwirkung sterilisiert. Nach Abschluss des Sterilisationsvorgangs wird der Datenträger in der zweiten Gehäusekammer angeordnet und diese verschlossen.

Ein weiteres Ausführungsbeispiel betrifft ein Verpackungssystem für Austauschkomponenten eines erfindungsgemäßen in-vivo Messsystems, umfassend ein Gehäuse mit mindesten zwei getrennten Kammern, mindestens einen Sensor zur Erzeugung von Messsignalen, die mit der zu messenden Analytkonzentration korrelieren, und einen Datenträger mit Kalibrationsdaten des mindestens einen Sensors, wobei der Sensor unter sterilen Bedingungen in einer ersten Kammer des Gehäuses und der Datenträger mit Kalibrationsdaten des Sensors in einer zweiten Kammer des Gehäuses angeordnet ist.

Weitere Einzelheiten und Vorteile der Erfindung werden anhand von Ausführungsbeispielen unter Bezugnahme auf die beigefügten Zeichnungen erläutert. Gleiche und einander entsprechende Komponenten sind dabei mit übereinstimmenden Bezugszeichen gekennzeichnet. Die im folgenden beschriebenen Merkmale können einzeln oder in Kombination zum Gegenstand von Ansprüchen gemacht werden. Es zeigen:
Fig. 1 eine schematische Darstellung einer Basisstation und eines daran angeschlossenen Sensors eines Systems zur in-vivo Messung einer Analytkonzentration in einem menschlichen oder tierischen Körper;
Fig. 2 die Basisstation des in Fig. 1 dargestellten Ausführungsbeispiels und ein Ausführungsbeispiels eines Verpackungssystems mit zum Anschluss an die Basisstation bestimmten Austauschkomponenten;
Fig. 3 das zum Anschluss der Austauschkomponenten an die Basisstation angesetzte Verpackungssystem;
Fig. 4 einen Arbeitsschritt zur Herstellung des in Fig. 2 dargestellten Verpackungssystems;
Fig. 5 ein weiterer Arbeitsschritt zur Herstellung des Verpackungssystems;
Fig. 6 das Verpackungssystem mit einer Umverpackung;
Fig. 7 ein weiteres Ausführungsbeispiel eines erfindungsgemäßen Systems zur in-vivo Messung einer Analytkonzentration in einem menschlichen oder tierischen Körper;
Fig. 8 eine Querschnittsdarstellung zu Figur 7; und
Fig. 9 ein Sensorgehäuse des in den Figuren 7 und 8 dargestellten Ausführungsbeispiels.

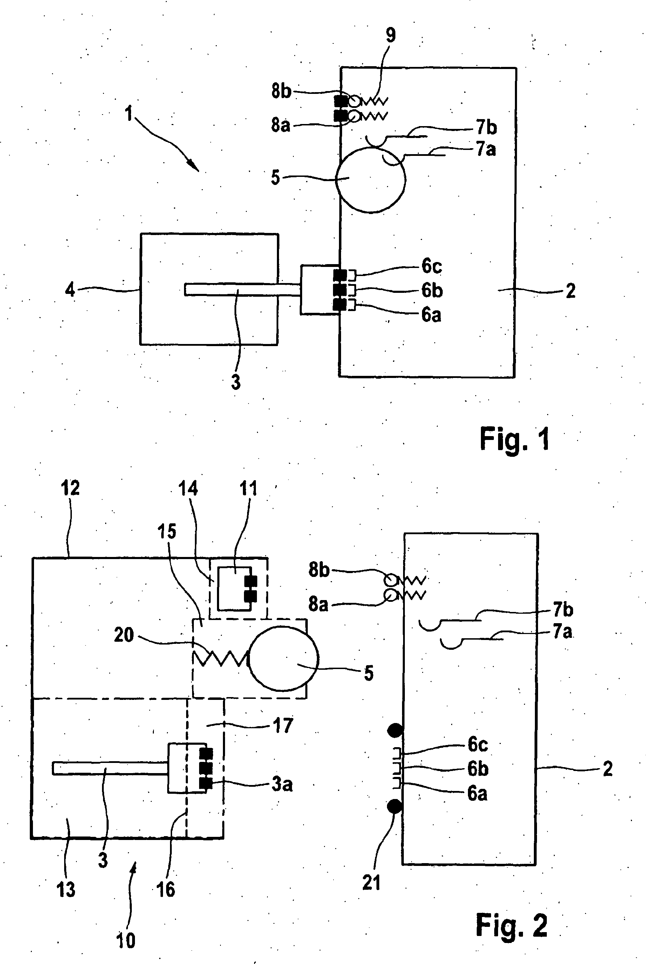

Fig. 1 zeigt in einer schematischen Darstellung eine Basisstation 2 eines Systems 1 zur in-vivo Messung einer Analytkonzentration in einem menschlichen oder tierischen Körper mit einem an die Basisstation 2 angeschlossenen Sensor 3 zur Erzeugung von Messsignalen, die mit der zu messenden Analytkonzentration korrelieren. In Fig. 1 ist ein menschlicher oder tierischer Körper, in den der Sensor 3 bei einer in-vivo Messung hineinragt, symbolisch durch den Kasten 4 dargestellt. Neben dem Sensor 3 gehört zu dem System 1 als weitere Verbrauchs- oder Austauschkomponente eine an die Basisstation 2 angeschlossene Batterie 5.

Die Basisstation 2 wird bestimmungsgemäß von einem Patienten währen der in-vivo Messungen am Körper getragen und enthält einen Potentiostaten, der den angeschlossenen elektrochemischen Sensor 3 mit Strom versorgt und das an einer Messelektrode des Sensors 3 anliegende elektrische Potential bezüglich einer Referenzelektrode des Sensors 3 auf einem vorgegeben Wert hält. Die Basisstation 2 enthält ferner elektronische Auswerteeinheit, die im Betrieb von einem angeschlossenen Sensor 3 erzeugte Messsignale mittels Kalibrationsdaten auswertet. Prinzipiell ist es jedoch auch möglich, die Auswerteeinheit in einem von der Basisstation separaten Gerät anzuordnen, dem die Messsignale, beispielsweise per Funk oder Datenleitung, zur Verfügung gestellt werden.

In Fig. 1 sind Anschlusskontakte 6a, 6b, 6c der Basisstation 2 zum Anschluss des Sensors 3 und Anschlusskontakte 7a, 7b der Basisstation 2 zum Anschluss der Batterie 5 dargestellt. Die Basisstation 1 hat ferner mindestens einen Dateneingang 8a, 8b zum Anschließen und Auslesen eines Datenträgers mit Kalibrationsdaten, der nach dem Auslesen der Kalibrationsdaten von der Basisstation 2 entfernt werden kann und deshalb in den in Fig. 1 dargestellten Betriebszustand nicht gezeigt ist. Der Dateneingang 8a, 8b ist mit Federelementen 9 gekoppelt, die durch Federkraft das Anschließen eines Datenträgers erleichtern. Bevorzugt handelt es sich bei dem Datenträger um einen Speicherchip, so dass der Dateneingang von elektrischen Anschlusskontakten gebildet wird. Der Datenträger kann beispielsweise auch ein magnetischer Datenträger sein und der Dateneingang 8a, 8b entsprechend einen Lesekopf umfassen.

In Fig. 2 ist die dargestellte Basisstation ohne angeschlossene Austauschkomponenten schematisch dargestellt. Zusätzlich zeigt Fig. 2 schematisch ein Verpackungssystem 10 für Austauschkomponenten (insbesondere Sensor 3, Batterie 5 und Datenträger 11 mit Kalibrationsdaten), die zusammen mit der Basisstation 2 ein System 1 zur in-vivo Messung einer Analytkonzentration in einem menschlichen oder tierischen Körper bilden. Das Verpackungssystem 10 umfasst ein Gehäuse 12 mit mindestens zwei getrennten Kammern 13, 14, 15, wobei in der ersten Kammer 13 unter sterilen Bedingungen der Sensor 3 und in einer zweiten Kammer 14 der Datenträger 11 mit Kalibrationsdaten des Sensors 3 angeordnet sind. Bei dem dargestellten Ausführungsbeispiel ist in einer dritten Kammer 15 die Batterie 5 angeordnet.

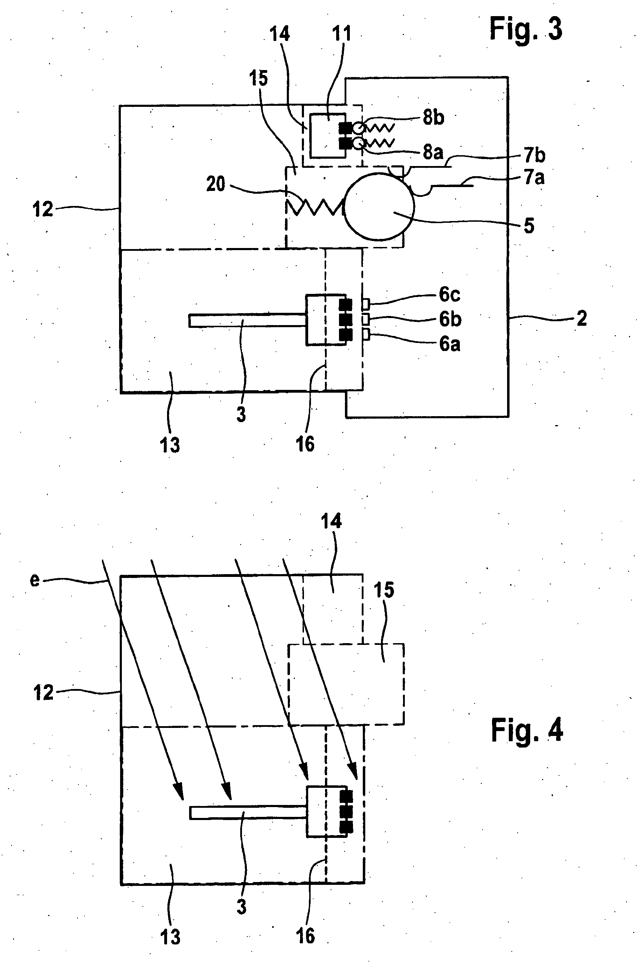

Das Gehäuse .12 ist derart an einer Schnittstelle der Basisstation 2 angepasst, dass der in dem Gehäuse 12 enthaltene Sensor 3 und der dazugehörende Datenträger 11 an die Basisstation 2 durch Ansetzen des Gehäuses 12 an die Schnittstelle anschließbar sind. In Fig. 3 ist schematisch das Ansetzen des Gehäuses 12 an die Schnittstelle der Basisstation 2 zum Anschließen der Austauschkomponenten 3, 5, 11 dargestellt. Durch Anschließen der Austauschkomponenten 3, 5, 11 wird das Messsystem 1 automatisch initialisiert und der Messvorgang gestartet.

Zum Anschließen der in dem Gehäuse 12 gelagerten Austauschkomponenten 3, 5, 11 wird das Gehäuse 12, insbesondere die sterile Gehäusekammer 13, geöffnet. Um das Öffnen zu erleichtern, ist das Gehäuse 12 des dargestellten Ausführungsbeispiels mit einer Sollbruchstelle 16 versehen, so dass ein die Kammern 13, 14, 15 verschließendes Gehäuseteil 17 von einem Benutzer leicht von dem Gehäuse 12 abgebrochen werden kann. Dieser abbrechbare Gehäuseteil 17 kann beispielsweise als Kappe ausgebildet sein. Bei dem dargestellten Ausführungsbeispiel verschließt der Gehäuseteil 17 sowohl die sterile Kammer 13, in der sich der Sensor 3 befindet, als auch die Kammern 14, 15, in denen sich der Datenträger 11 und die Batterie 5 befinden. Möglich ist es aber auch, diese Kammern 13, 14, 15 durch separate Gehäuseteile zu verschließen, die einzeln entfernt werden müsse. Insbesondere zum Verschließen von nicht-sterilen Kammern, beispielsweise der den Datenträger 7 oder die Batterie 5 enthaltenden Kammern 14, 15, kann auch eine abziehbare Folie oder ähnliches verwendet werden.

Das Gehäuse 12 enthält ein Federelement 20, dass beim Ansetzen des Gehäuses 12 an die Schnittstelle der Basisstation 2 das Anschließen der Batterie 5 unterstützt. In entsprechender Weise können auch in der ersten Kammer 13 und in der zweiten Kammer 14 Federelemente angeordnet sein, um das Anschließen des Sensors 3 und/oder des Datenträgers 11 an die Basisstation zu unterstützen.

Bei dem dargestellten Ausführungsbeispiel sind das Gehäuse 12 und die Schnittstelle der Basisstation 2 derart aufeinander abgestimmt, dass beim Ansetzen des Gehäuses 12 an die Schnittstelle zunächst die Batterie 5 und der Datenträger 11 an die Basisstation 2 angeschlossen werden und erst danach der Sensor 3 an die Basisstation 2 über die dafür vorgesehenen Kontakte 6a, 6b, 6c angeschlossen wird. Bei dem dargestellten Ausführungsbeispiel hat der Sensor 3 eine flache Struktur und wird mit einem Nullkraftstecker 3a an die Basisstation 2 angeschlossen. Der Sensor 3 kann beispielsweise auch eine Sandwichstruktur haben oder rotationssymmetrisch aufgebaut sein und die Kontakte 6a, 6b, 6c dazu passen ausgestaltet sein.

Eine Dichtung 21 der Basisstation 2, die bei dem darstellten Ausführungsbeispiel als Dichtring ausgeführt ist, sorgt dabei für eine wasserdichte Ankopplung des Sensors 3 an die Basisstation 2, so dass keine Feuchtigkeit in den Innenraum der Basisstation 2 eindringen kann. Die Basisstation 2 kann somit beispielsweise auf dem Bauch eines Patienten getragen werden, ohne durch Körperflüssigkeiten beschädigt zu werden. Die Dichtung 21 bewirkt eine hochohmige Abdichtung der Basisstation 2 und des angeschlossenen Sensors 3. Auf diese Weise kann der Sensor 3 als elektrochemischer Sensor ohne Beeinträchtigung durch Leckströme mittels eines in der Basisstation 2 angeordneten Potentiostaten mit Spannung versorgt werden.

Das Gehäuse 12 ist bei dem dargestellten Ausführungsbeispiel als Blisterverpackung ausgebildet. In das Kunststoffformteil der Blisterverpackung sind Fächer eingeformt, welche den Boden und die Wände der Kammern 13, 14, 15 des Gehäuses 12 bilden. In einem ersten Arbeitsschritt, der in Fig. 4 dargestellt ist, wird in der ersten Gehäusekammer 13 ein Sensor 3 angeordnet und die Kammer 13 versiegelt. Anschließend wird der Sensor 3 in der Gehäusekammer 13 durch Strahlungseinwirkung sterilisiert. Besonders geeignet sind Elektronenstrahlen e mit einer Dosis von mindestens 20 kGy. Geeignet ist insbesondere eine Elektronenstrahldosis von 28 kGy.

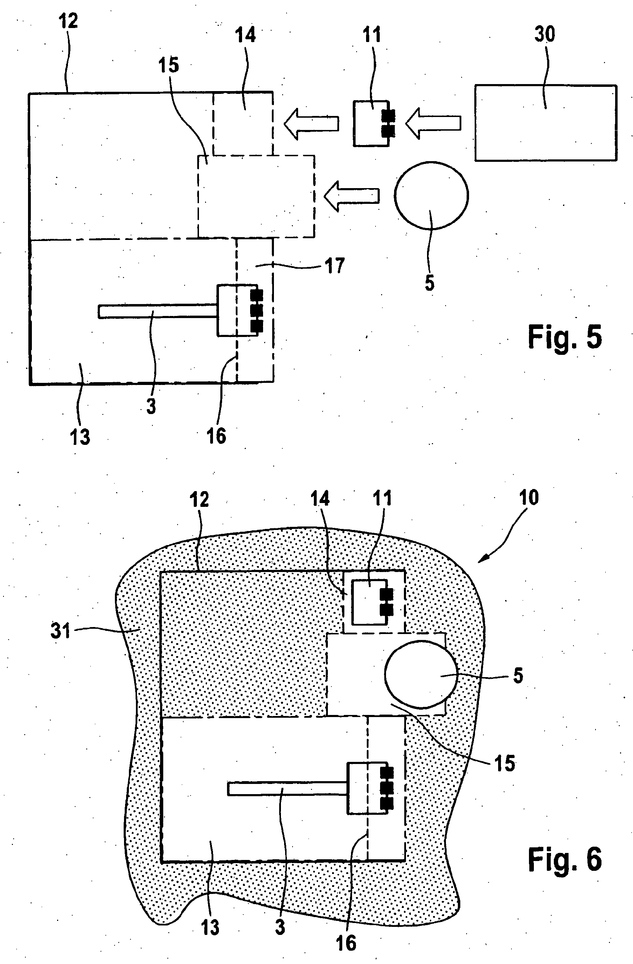

In einem weiteren Arbeitsschritt, der in Fig. 5 dargestellt ist, wird der Datenträger 11 mit Kalibrationsdaten 30 des in der ersten Gehäusekammer 13 angeordneten Sensors 3 beschrieben. Diese Kalibrationsdaten 30 werden durch Stichproben an Sensoren 13 derselben Produktionscharge nach Abschluss des Sterilisationsvorgangs ermittelt. Danach werden der Datenträger 11 in der zweiten Gehäusekammer 14 sowie die Batterie 5 in der Gehäusekammer 15 angeordnet und die Gehäusekammer 14, 15 verschlossen. Die Gehäusekammern 13, 14, 15 können beispielsweise mittels Kunststoff- oder Metallfolie in der für Blisterverpackungen üblichen Weise versiegelt werden.

In einem letzten Arbeitsschritt wird das nun fertige Verpackungssystem 10 in eine Umverpackung, in der es in den Handel gebracht wird, eingepackt, beispielsweise in Kunststofffolie eingeschweißt. Ein derartiges Verpackungssystem 10 mit Umverpackung 31 ist in. Fig. 6 dargestellt.

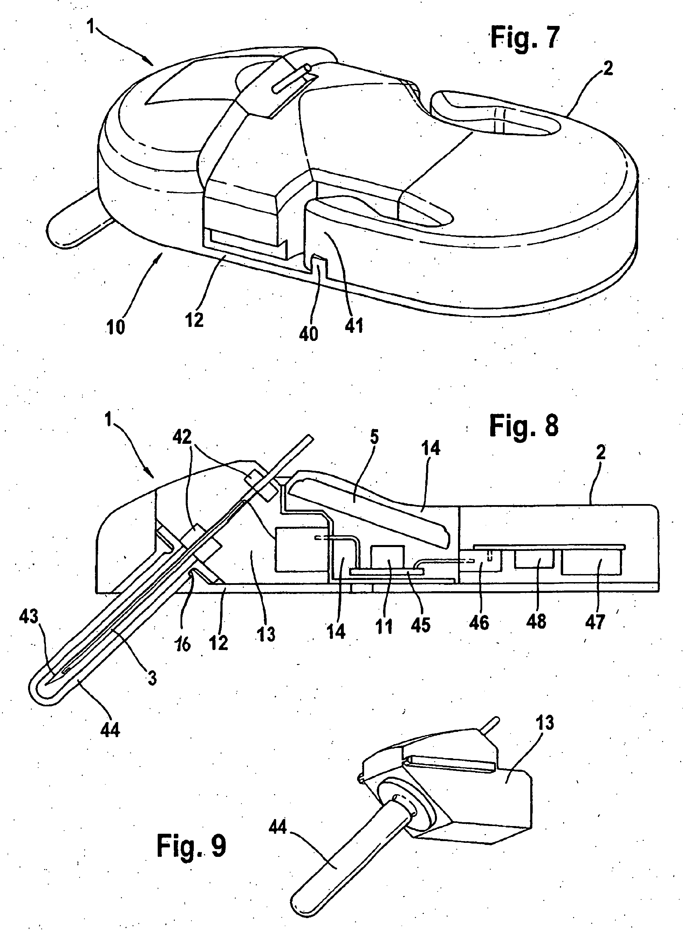

Bei dem im Vorhergehenden anhand der Figuren 1 bis 6 erläuterten Ausführungsbeispiel ist das die Austauschkomponenten enthaltende Gehäuse 12 ein Verpackungsgehäuse, das bestimmungsgemäß vor der Durchführung einer in-vivo Messung wieder von der Basisstation 2 abgenommen wird. Im Folgenden wird anhand der Figuren 7 bis 9 ein weiteres Ausführungsbeispiel erläutert, bei dem das die Austauschkomponenten enthaltende Gehäuse 12 während der Durchführung von in-vivo Messungen mit der Basisstation 2 verbunden bleibt.

Fig. 7 zeigt in einer Schrägansicht die Basisstation 2 mit daran angeschlossenem Gehäuse 12, das Austauschkomponenten des Systems 1 enthält. Fig. 8 zeigt eine Querschnittsansicht zu Fig. 7, in der die sterile Gehäusekammer 13 mit dem darin angeordneten Sensor 3 sowie die zweite Gehäusekammer 14 mit darin angeordneter Batterie 5 und Datenträger 11, auf den Kalibrationsdaten des Sensors 3 gespeichert sind. Die Basisstation 2 enthält einen Potentiostaten 48 zur Strom- und Spannungsversorgung des Sensors 3 und eine als Mikroprozessor ausgebildete Auswerteeinheit 47, die im Betrieb von dem angeschlossenen Sensor 3 erzeugten Messsignale mittels der Kalibrationsdaten, die von dem zu dem angeschlossenen Sensor 3 gehörenden Datenträger 11 gelesen wurden, auswertet. Prinzipiell kann der Potentiostat 48 jedoch auch als Verbrauchskomponente ausgebildet und zusammen mit dem Sensor 3 in dem Gehäuse 12 angeordnet werden, so dass an eine Abdichtung der Basisstation 2 hinsichtlich Hochohmigkeit geringere Anforderungen gestellt werden können. Ferner kann die Auswerteeinheit 47 in einem von der Basisstation 2 getrennten Gerät angeordnet werden, dem Daten von der Basisstation 2 zur Verfügung gestellt werden.

Das Gehäuse 10 des Verpackungssystems für Verbrauchskomponenten ist ebenso wie das Gehäuse der Basisstation 2 aus Hartplastik gefertigt. Die Schnittstelle der Basisstation 2 und das die Verbrauchskomponenten enthaltende Gehäuse 12 sind bei dem dargestellten Ausführungsbeispiel für eine formschlüssige Verbindung ausgelegt. Das Gehäuse 12 weist Rastzapfen 40 auf, die in dazu passende Ausnehmungen der Schnittstelle der Basisstation 2 eingreifen. Diese Ausnehmungen sind an den Außenseiten von zwei Federschenkeln 41 angeordnet, so dass die Rastzapfen durch Federkraft in die Ausnehmungen gedrückt werden. Die Federschenkel 41 können zusammengedrückt werden, so dass sich die Rastzapfen 40 des die Verbrauchskomponenten enthaltenen Gehäuses 12 aus den dazu passenden Ausnehmungen lösen und das Gehäuse 12 von der Basisstation 2 abgenommen werden kann. In entsprechender Weise kann bei zusammengepressten Federschenkeln 41 das die Verbrauchskomponenten enthaltende Gehäuse 12 an die Basisstation 2 angesetzt werden.

Alternativ oder zusätzlich zu einer formschlüssigen Verbindung kann das die Verbrauchskomponenten enthaltende Gehäuse auch so ausgebildet werden, dass es zum Anschließen des in dem Gehäuse enthaltenen Sensors klemmschlüssig mit der Basisstation 2 verbindbar ist.

Die in Fig. 8 dargestellte Querschnittsansicht zeigt, dass das Gehäuse 12 zwei getrennte Kammern 13, 14 aufweist, wobei in einer ersten Kammer 13 unter sterilen Bedingungen der Sensor 3 und in der zweiten Kammer 14 ein Datenträger 11 mit Kalibrationsdaten des Sensors 3 und eine Batterie 5 zur Stromversorgung der Basisstation 2 angeordnet sind. Anschlussleitungen des Sensors 3 führen aus der ersten Kammer 13 in die zweite Kammer 14 zu einer Leiterplatte 45, die mit dem als Speicherchip ausgeführten Datenträger 11 kontaktiert ist. Die Leiterplatte 45 ist über eine Steckverbindung 46, im dargestellten Ausführungsbeispiel eine mehrpolige Steckverbindung, an die Basisstation 2 angeschlossen.

Die sterile Kammer 13, in welcher der Sensor 3 gelagert ist, ist von zwei Septen 42 verschlossen, wobei eine Insertionsnadel 43 zum Einführen des Sensors 3 in einen menschlichen oder tierischen Körper durch die Septen 42 hindurchgeführt ist. Das aus der Gehäusekammer 13 herausragende Vorderende der Insertionsnadel 43 ist von einer Sterilschutzkappe 44 umgeben, die erst abgenommen wird, wenn der Sensor 3 mittels der Insertionsnadel 43 in einen menschlichen oder tierischen Körper eingeführt werden soll. Die Sterilschutzkappe 44 ist bei dem dargestellten Ausführungsbeispiel über eine Sollbruchstelle 16 mit dem übrigen Gehäuse 12 verbunden.

Zur Insertion des Sensors 2 wird das aus dem Verpackungssystem und der Basisstation 2 zusammengefügte System 1 beispielsweise auf dem Bauch eines Patienten angeordnet und die Insertionsnadel 43 in den Körper des Patienten eingestochen. Anschließend kann die Insertionsnadel 43, die beispielsweise als eine den Sensor 3 tragende Rinne ausgeführt sein kann, wieder aus dem Körper des Patienten herausgezogen, wobei der Sensor 3 im Körper des Patienten verbleibt.

Zum Verpacken des Sensors 3 und eines Datenträgers 11, auf dem Kalibrationsdaten des Sensors 3 gespeichert sind, wird der Sensor 3 zunächst in der ersten Gehäusekammer 13 angeordnet und diese versiegelt. Zur Herstellung des in Fig. 8 dargestellten Ausführungsbeispiels wird bei diesem Arbeitsschritt auch eine Sterilschutzkappe 44 um das aus der ersten Gehäusekammer 13 herausragende Ende des Sensors 3 sowie die den Sensor 3 tragende Insertionsnadel 43 angeordnet und die Sterilschutzkappe 44 mit der Gehäusekammer 13 verbunden. Anschließend wird die Gehäusekammer 13 intensiver Elektronenstrahlung ausgesetzt, so dass der Sensor 3 und die Insertionsnadel 43 sterilisiert werden. In Fig. 9 ist eine Detailansicht der ersten Gehäusekammer 13 mit der daran angebrachten Sterilschutzkappe 44 dargestellt, die nach Einbringen des Sensors 3 zusammen durch Bestrahlen sterilisiert werden.

In einem weiteren Arbeitsschritt wird die erste Gehäusekammer 13 mit der zweiten Gehäusekammer 14 zusammengefügt, um das die Verbrauchskomponenten enthaltende Gehäuse 12 und damit das im Vorhergehenden beschriebene Verpackungssystem 10 für Verbrauchskomponenten des Messsystems 1 zu schaffen.

### Bezugszeichenliste

- 1: System zur in-vivo Messung einer Analytkonzentration
- 2: Basisstation
- 3: Sensor
- 4: Menschlicher Körper
- 5: Batterie
- 6a, 6b, 6c: Anschlusskontakte
- 7a, 7b: Anschlusskontakte
- 8a, 8b: Dateneingang der Basisstation
- 9: Federelement
- 10: Verpackungssystem für Verbrauchskomponenten des Messsystems
- 11: Datenträger
- 12: Verbrauchskomponenten enthaltendes Gehäuse
- 13: Gehäusekammer
- 14: Gehäusekammer
- 15: Gehäusekammer
- 16: Sollbruchstelle
- 17: Gehäuseteil
- 20: Federelement
- 30: Kalibrationsdaten
- 31: Umverpackung
- 40: Rastzapfen
- 41: Federschenkel der Basisstation 2
- 42: Septa
- 43: Insertionsnadel
- 44: Sterilschutzkappe
- 45: Leiterplatte
- 46: Steckverbindung
- 47: Auswerteeinheit
- 48: Potentiostat

## Patentansprüche

1. Gehäuse für Verbrauchskömponenten eines in-vivo Messsystems (1), wobei das Gehäuse (12) mindestens zwei getrennte Kammern (13, 14, 15) aufweist,
in einer versiegelten ersten Kammer (13) des Gehäuses (12) als erste Komponente ein Sensor (3) zur Erzeugung von Messsignalen, die mit der zu messenden Analytkonzentration korrelieren, unter sterilen Bedingungen angeordnet ist, und
in einer zweiten Kammer (14) des Gehäuses (12) als zweite Komponente ein Datenträger (11), auf dem Kalibrationsdaten des Sensors (3) gespeichert sind, angeordnet ist,
wobei der Sensor (3) und der Datenträger (11) an eine Basisstation (2) des in-vivo Messsystems (1) anschließbar sind, so dass im Betrieb von dem Sensor (3) erzeugte Messsignale an eine Auswerteeinheit (47) übermittelt werden, welche die von dem angeschlossenen Sensor (3) erzeugten Messsignale mittels der Kalibrationsdaten, die von dem Datenträger (11) gelesen wurden, auswertet.
**dadurch gekennzeichnet, dass**
das Gehäuse (12) an eine Schnittstelle der Basisstation (2), die mindestens einen Dateneingang (8a, 8b) zum Anschließen und Auslesen des Datenträgers (11) und Anschlusskontakte (6a, 6b, 6c) für den Sensor (3) hat, angepasst ist, so dass der in dem Gehäuse (12) enthaltene Sensor (3) und der dazugehörende Datenträger (11) an die Basisstation (2) durch Ansetzen des Gehäuses (12) an das Gehäuse der Basisstation (2) arischließbar sind.

2. Gehäuse nach Anspruch 1, **dadurch gekennzeichnet, dass** das Gehäuse (12) als weitere Verbrauchskomponente eine Batterie (5) zur Stromversorgung der Basisstation (2) enthält, die an die Basisstation (2) durch Ansetzen des Gehäuses (12) an die Schnittstelle anschließbar ist.

3. Gehäuse nach einem der vorstehenden Ansprüche, **dadurch gekennzeichnet, dass** das Gehäuse (12) eine Sollbruchstelle (17) zum Entfernen eines Gehäuseteils (17, 44), der die den Sensor (3) enthaltende Kammer (13) verschließt, aufweist.

4. System zur in-vivo Messung einer Analytkonzentration in einem menschlichen oder tierischen Körper, umfassend
ein Gehäuse (12) nach einem der vorstehenden Ansprüche, das einen Sensor (3) und einen Datenträger (11), auf dem Kalibrationsdaten des Sensors (3) gespeichert sind, enthält, und
eine Basisstation (2), an die jeweils mindestens ein solcher austauschbarer Sen- , sor (3) und ein dazugehörender Datenträger (11) mit Kalibrationsdaten anschließbar sind, so dass im Betrieb von einem angeschlossenen Sensor (3) erzeugte Messsignale an eine Auswerteeinheit (47) übermittelt werden, welche die von dem angeschlossenen Sensor (3) erzeugten Messsignale mittels der Kalibrationsdaten, die von dem zu dem angeschlossenen Sensor (3) gehörenden Datenträger (11) gelesen wurden, auswertet, wobei
die Basisstation (2) Anschlusskontakte (6a, 6b, 6c) für den Sensor (3) und mindestens einen Dateneingang (8a, 8b) zum Anschließen und Auslesen eines Datenträgers (11) mit Kalibrationsdaten hat.

5. System nach Anspruch 4, **dadurch gekennzeichnet, dass** der Sensor (3) ein elektrochemischer Sensor ist, der im Betrieb an einen in der Basisstation (2) angeordneten Potentiostaten (48) angeschlossen ist.

6. System nach Anspruch 4 oder 5, **dadurch gekennzeichnet, dass** die Auswerteeinheit (47) in der Basisstation (2) angeordnet ist.

7. System nach einem der Ansprüche 4 bis 6, **dadurch gekennzeichnet, dass** das Gehäuse (12) zum Anschließen des darin enthaltenen Sensors (3) klemm- oder formschlüssig mit der Bäsisstation (2) verbindbar ist.

8. System nach einem der Ansprüche 4 bis 7, **dadurch gekennzeichnet, dass** das Gehäuse (12) bestimmungsgemäß während der Durchführung einer in-vivo Messung mit der Basisstation (2) verbunden bleibt.

9. System nach einem der Ansprüche 4 bis 7, **dadurch gekennzeichnet, dass** das Gehäuse (12) ein Verpackungsgehäuse ist, das bestimmungsgemäß vor dem
Durchführen einer in-vivo Messung ganz oder teilweise wieder von der Basisstation (2) abgenommen wird.

10. System nach einem der Ansprüche 4 bis 9, **dadurch gekennzeichnet, dass** das Gehäuse (12) und/oder die Basisstation (2) ein Federelement (9,20) enthalten, das beim Ansetzen des Gehäuses (12) an die Schnittstelle der Basisstation (2) das Anschließen des in dem Gehäuse (12) enthaltenen Sensors (3) und/oder des Datenträgers (11) an die Basisstation (2) unerstützt.

11. System nach einem der Ansprüche 4 bis 10, **dadurch gekennzeichnet, dass** das Anschließen in dem Gehäuse (12) enthaltenen Komponenten (3, 5, 7) an die Basisstation (2) eine Inbetriebnahme des Systems (1), insbesondere das Erzeugen von Messsignalen und deren Auswertung bewirkt.

## Claims

1. Housing for consumable components of an in-vivo measurement system, wherein the housing has at least two separate chambers (13,14,15),
a sensor (3) for providing measurement signals, which correlate with the analyte concentration to be measured, is arranged as a first component under sterile conditions in a sealed first chamber (13) of the housing (12), and
a data carrier (11), on which calibration data of the sensor (3) are stored, is arranged as a second component in a second chamber (14) of the housing (12),
wherein the sensor (3) and the data carrier (11) can be connected to a base station (2) of the in-vivo measurement system so that in operation measurement signals created by the sensor (3) are transmitted to an evaluation unit (47) that evaluates the measuring signals generated by the connected sensor (3) by means of the calibration data that were read from the data carrier (11),
**characterized in that**
the housing is adapted to an interface of the base station (2), which has at least one data port for connecting to and reading of the data carrier (11) and contacts for connecting the sensor (3) to, such that the sensor (3) contained in the housing (12) and the therewith associated data carrier (11) can be connected to the base station (2) by the setting of the housing (12) to the housing of the base station (2).

2. Housing according to claim 1, **characterized in that** the housing (12) contains, as another consumable component, a battery (5) for the power supply of the base station (2), which battery can be connected to the base station (2) by the setting the housing (12) to the interface.

3. Housing according to any of above claims, **characterized in that** the housing (12) is provided with a rupture joint (17) for the removal of a housing part (17, 44) that seals the chamber (13) containing the sensor (3).

4. System for in-vivo measurement of an analyte concentration in a human or animal body, comprising
a housing according to any of above claims, which contains a sensor (3) and a data carrier (11) on which calibration data of the sensor (3) are stored, and
a base station (2), to which such an exchangeable sensor and a data carrier belonging to it can be connected such that in operation measurement signals created by a connected sensor (3) are transmitted to an evaluation unit (47) that evaluates the measuring signals generated by the connected sensor (3) by means of the calibration data that were read from the data carrier (11), wherein
the base station (2) comprises contacts (6a, 6b, 6c) for the sensor (3) and at least one data port (8a, 8b) for connecting to and reading of a data carrier (11) with calibration data.

5. System according to claim 4, **characterized in that** the sensor (3) is an electrochemical sensor which is in operation connected to a potentiostat (48) arranged in the base station (2).

6. System according to claim 4 or 5, **characterized in that** the evaluation unit (47) is arranged in the base station (2).

7. A system according to any of claims 4 to 6, **characterized in that**, to connect the therein contained sensor (3), the housing (12) can be fastened to the base station (2) in an interlocking or clamping manner.

8. A system according to any of claim 4 to 7, **characterized in that** the housing (12) is configured to be attached to the base station (2) during the carrying out of the in-vivo measurement.

9. A system according to any of claims 4 to 8, **characterized in that** the housing (12) is a packaging housing that is configured to be either totally or partially removed from the base station (2) prior to the carrying out of an in-vivo measurement.

10. A system according to any of claims 4 to 9, **characterized in that** the housing (12) and/or the base station (2) are provided with a spring element (9, 20) that, upon setting the housing (12) to the interface of the base station (2), facilitates connecting of the sensor (3) and/or the data carrier (11) to the base station (2).

11. A system according to any of claims 4 to 10, **characterized in that** the connecting of the components (3, 5, 7), contained in the housing (12), to the base station (2) causes initiation of the system (1), in particular the generation of measuring signals and their evaluation.

## Revendications

1. Boîtier pour des composants consommables d'un système de mesure *in vivo* (1), dans lequel le boîtier (12) présente au moins deux chambres distinctes (13, 14, 15), un capteur (3) comme premier composant pour la production de signaux de mesure, qui sont en corrélation avec la concentration d'analyte à mesurer, est disposé dans des conditions stériles dans une première chambre scellée (13) du boîtier (12), et un support de données (11) est disposé comme deuxième composant dans une deuxième chambre (14) du boîtier (12), sur lequel des données de calibrage du capteur (3) sont mémorisées, dans lequel le capteur (3) et le support de données (11) peuvent être connectés à une station de base (2) du système de mesure *in vivo* (1), de telle manière qu'en fonctionnement des signaux de mesure produits par le capteur (3) soient transmis à une unité d'exploitation (47), qui exploite les signaux de mesure produits au capteur connecté (3) au moyen des données de calibrage, qui ont été lues du support de données (11), **caractérisé en ce que** le boîtier (12) est adapté à une interface de la station de base (2), qui comporte au moins une entrée de données (8a, 8b) pour la connexion et la lecture du support de données (11) et des contacts de raccordement (6a, 6b, 6c) pour le capteur (3), de telle manière que le capteur (3) contenu dans le boîtier (12) et le support de données (11) correspondant puissent être connectés à la station de base (2) par la pose du boîtier (12) sur le boîtier de la station de base (2).

2. Boîtier selon la revendication 1, **caractérisé en ce que** le boîtier (12) contient comme autre composant consommable une batterie (5) pour l'alimentation électrique de la station de base (2), qui peut être connectée à la station de base (2) par la pose du boîtier (12) sur l'interface.

3. Boîtier selon l'une quelconque des revendications précédentes, **caractérisé en ce que** le boîtier (12) présente une zone de rupture (17) pour l'enlèvement d'une partie de boîtier (17, 44), qui ferme la chambre (13) contenant le capteur (3).

4. Système pour la mesure *in vivo* d'une concentration d'un analyte dans un corps humain ou animal, comprenant
un boîtier (12) selon l'une quelconque des revendications précédentes, qui contient un capteur (3) et un support de données (11), sur lequel des données de calibrage du capteur (3) sont mémorisées, et
une station de base (2), à laquelle respectivement au moins un tel capteur remplaçable (3) et un support de données correspondant (11) avec des données de calibrage peuvent être connectés, de telle manière qu'en fonctionnement des signaux de mesure produits par un capteur connecté (3) soient transmis à une unité d'exploitation (47), qui exploite les signaux de mesure produits par le capteur connecté (3) au moyen de données de calibrage, qui ont été lues du support de données (11) correspondant au capteur connecté (3), dans lequel
la station de base (2) comporte des contacts de raccordement (6a, 6b, 6c) pour le capteur (3) et au moins une entrée de données (8a, 8b) pour la connexion et la lecture d'un support de données (11) avec des données de calibrage.

5. Système selon la revendication 4, **caractérisé en ce que** le capteur (3) est un capteur électrochimique, qui est connecté en fonctionnement à un potentiostat (48) disposé dans la station de base (2).

6. Système selon la revendication 4 ou 5, **caractérisé en ce que** l'unité d'exploitation (47) est disposée dans la station de base (2).

7. Système selon l'une quelconque des revendications 4 à 6, **caractérisé en ce que** le- boîtier (12) peut être assemblé par serrage ou emboîtement à la station de base (2) pour la connexion du capteur (3) qu'il contient.

8. Système selon l'une quelconque des revendications 4 à 7, **caractérisé en ce que** le boîtier (12) reste assemblé à la station de base (2) comme prévu pendant l'exécution d'une mesure *in vivo.*

9. Système selon l'une quelconque des revendications 4 à 7, **caractérisé en ce que** le boîtier (12) est un boîtier d'emballage, qui est de nouveau enlevé totalement ou partiellement de la station de base (2) comme prévu avant l'exécution d'une mesure in vivo.

10. Système selon l'une quelconque des revendications 4 à 9, **caractérisé en ce que** le boîtier (12) et/ou la station de base (2) contiennent un élément de ressort (9, 20), qui aide la connexion du capteur (3) contenu dans le boîtier (12) et/ou du support de données (11) à la station de base (2) lors de la pose du boîtier (12) sur l'interface de la station de base (2).

11. Système selon l'une quelconque des revendications 4 à 10, **caractérisé en ce que** la connexion des composants (3, 5, 7) contenus dans le boîtier (12) à la station de base (2) provoque une mise en service du système (1), en particulier la production de signaux de mesure et leur exploitation.
